# EUROPEAN PATENT APPLICATION

(11) **EP 4 471 008 A1**
(43) Date of publication of application: **04.12.2024**
(21) Application number: 23747047.1
(22) Date of filing: 26.01.2023
(51) Int. Cl.: C07C 319/08, C07C 321/14, C08G 18/38, G02B 3/00, G02C 7/02

(54) **POLYTHIOL COMPOSITION AND APPLICATION OF SAME**

(30) Priority: 27.01.2022 JP 2022010749
(71) Applicant: MITSUI CHEMICALS, INC., Tokyo 104-0028 (JP)
(72) Inventor: NAKAI, Shinnosuke, Omuta-shi, Fukuoka 836-8610 (JP)
(74) Representative: Mewburn Ellis LLP
(86) International application number: PCT/JP2023/002503
(87) International publication number: WO 2023/145837

(57) **Abstract**

A polythiol composition, comprising a polythiol compound, wherein: the polythiol composition comprises a compound C2 that has a retention time of from 16.0 minutes to 19.0 minutes in a high-performance liquid chromatography measurement performed under a specific measurement conditions A, and in the high-performance liquid chromatography measurement, the compound C2 has a peak area of 1.000 or more with respect to a total of 100 of a peak area of the compound C2 and a peak area of a main component of the polythiol composition.

## Description

### Technical Field

The present disclosure relates to a polythiol composition, and an application thereof.

### Background Art

Plastic lenses, which are resin-containing lenses, are lightweight, hardly breakable, and dyeable as compared to inorganic lenses; therefore, in recent years, the use of plastic lenses as eyeglass lenses, camera lenses, and the like has been rapidly increased.

For example, various studies have been conducted on thiourethane resin-containing lenses (see, for example, Patent Documents 1 to 3).
Patent Document 1: Japanese Patent Application Laid-Open (JP-A) No. S63-46213
Patent Document 2: JP-A No. H2-270859
Patent Document 3: JP-A No. H7-252207

### SUMMARY OF THE INVENTION

### Technical Problem

A thiourethane resin is usually produced using a polythiol composition and a polyisocyanate compound as raw materials.

For such a thiourethane resin, it may be demanded to further improve the dyeability.

An object of one aspect of the disclosure is to provide: a polythiol composition from which a thiourethane resin having excellent dyeability can be produced; and an application thereof.

### Solution to Problem

Means for solving the above-described problems encompass the following aspects.
<1> A polythiol composition, comprising a polythiol compound,
   wherein:
   the polythiol composition comprises a compound C2 that has a retention time of from 16.0 minutes to 19.0 minutes in a high-performance liquid chromatography measurement performed under the following measurement conditions A, and
   in the high-performance liquid chromatography measurement, the compound C2 has a peak area of 1.000 or more with respect to a total of 100 of a peak area of the compound C2 and a peak area of a main component of the polythiol composition:
      -Measurement Conditions A-
      MIGHTYSIL (registered trademark) RP-18 GP manufactured by Kanto Chemical Co., Inc. (particle size S: 5 µm, column shape: Φ6 mm × 150 mm, product number: 25477-96) is used as a column;
      a mixed solution of acetonitrile/0.01 mol/L-aqueous potassium dihydrogen phosphate solution = 60/40 (vol/vol) is used as a mobile phase;
      a mixed solution of 160 mg of the polythiol composition and 10 mL of acetonitrile is used as a measurement solution;
      a UV detector having a measurement wavelength of 230 nm is used as a detector;
      a column temperature is set at 40°C;
      a flow rate is set at 1.0 mL/min; and
      an injection amount is set at 2 µL.
<2> The polythiol composition according to <1>, wherein the compound C2 is a polythiol compound having a molecular weight of 320.
<3> The polythiol composition according to <1> or <2>,
   wherein:
   the polythiol composition further comprises a compound C3 that has a retention time of from 32.0 minutes to 35.0 minutes in the high-performance liquid chromatography measurement, and
   in the high-performance liquid chromatography measurement, the compound C3 has a peak area of 0.050 or more with respect to a total of 100 of a peak area of the compound C3 and the peak area of the main component of the polythiol composition.
<4> The polythiol composition according to <3>, wherein the compound C3 is a polythiol compound having a molecular weight of 426.
<5> The polythiol composition according to any one of <1> to <4>,
   wherein:
   the polythiol composition further comprises a compound C4 that has a retention time of from 21.5 minutes to 25.0 minutes in the high-performance liquid chromatography measurement, and
   in the high-performance liquid chromatography measurement, the compound C4 has a peak area of 0.030 or more with respect to a total of 100 of a peak area of the compound C4 and the peak area of the main component of the polythiol composition.
<6> The polythiol composition according to <5>, wherein the compound C4 is a polythiol compound having a molecular weight of 380.
<7> The polythiol composition according to any one of <1> to <6>, wherein the main component is a polythiol compound (XA) comprising three or more mercapto groups.
<8> The polythiol composition according to <7>, wherein the polythiol compound (XA) is a polythiol component A1 that is 4-mercaptomethyl-1,8-dimercapto-3,6-dithiaoctane, or a polythiol component A2 that is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.
<9> The polythiol composition according to <7>, wherein polythiol compound (XA) is a polythiol component A1 that is 4-mercaptomethyl-1,8-dimercapto-3,6-dithiaoctane.
<10> A polymerizable composition for an optical material, comprising:
   a polyiso(thio)cyanate compound; and
   the polythiol composition according to any one of <1> to <9>.
<11> The polymerizable composition for an optical material according to <10>, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane,2,6-bis(isocyanatomethyl)bicyclo-[2.2.1]-heptane, tolylene diisocyanate, 4,4'-diphenylmethane diisocyanate, and phenylene diisocyanate.
<12> The polymerizable composition for an optical material according to <10> or <11>, comprising a polyiso(thio)cyanate composition comprising the polyiso(thio)cyanate compound.
<13> The polymerizable composition for an optical material according to <12>, wherein:
   the polyiso(thio)cyanate composition comprises:
   xylylene diisocyanate; and
   at least one selected from the group consisting of the following compounds (N1), (N2), and (N3),
   when the polyiso(thio)cyanate composition comprises the compound (N1),the compound (N1) has a peak area of 0.20 ppm or more with respect to a peak area of xylylene diisocyanate of 1 in a gas chromatography measurement performed under the following GC conditions 1,
   when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
   when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
      -GC Conditions 1-
         Filler: DB-1 (film thickness) 1.5 µm
         Column: 0.53 mm in inner diameter × 60 m in length (manufactured by Agilent Technologies, Inc.)
         Oven temperature: increased from 130°C to 220°C at 3°C/min, and further increased to 300°C at 10°C/min after reaching 220°C
         Split ratio: pulsed splitless method
         Inlet temperature: 280°C
         Detector temperature: 300°C
         Carrier gas: N₂ 158 kPa, H₂ 55 kPa, Air 45 kPa (constant pressure control)
         Solvent: chloroform
         Sample concentration: 2.0%-by-mass chloroform solution
         Injection amount: 2 µL
         Detection method: FID
      -GC Conditions 2-
         Column: HP-50+, 0.25 mm in inner diameter × 30 m in length × 0.25 µm in film thickness (manufactured by Hewlett-Packard Company)
         Oven temperature: increased from 50°C to 280°C at 10°C/min, and held for 6 minutes after reaching 280°C
         Split ratio: pulsed splitless method
         Inlet temperature: 200°C
         Detector temperature: 280°C
         Carrier gas: He
         Carrier gas flow rate: 1.0 ml/min (constant flow rate control)
         Sample concentration: 1.0%-by-mass dichloromethane solution
         Injection amount: 1.0 µL
         Detection method: SIM (monitoring ion: m/z 180, 215) (content ratio of xylylene diisocyanate) 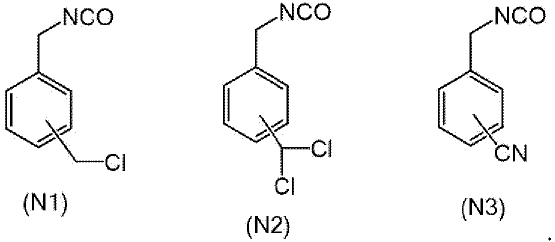
<14> A resin, which is a cured product of the polymerizable composition for an optical material according to any one of <10> to <13>.
<15> A formed body, comprising the resin according to <14>.
<16> An optical material, comprising the resin according to <14>.
<17> A lens, comprising the resin according to <14>.
<18> A method of producing a polymerizable composition for an optical material, the method comprising the step of mixing a polyiso(thio)cyanate composition comprising a polyiso(thio)cyanate compound with the polythiol composition according to any one of <1> to <9>.
<19> The method of producing a polymerizable composition for an optical material according to <18>, wherein:
   the polyiso(thio)cyanate composition comprises:
   xylylene diisocyanate; and
   at least one selected from the group consisting of the following compounds (N1), (N2), and (N3),
   when the polyiso(thio)cyanate composition comprises the compound (N1), the compound (N1) has a peak area of 0.20 ppm or more with respect to a peak area of xylylene diisocyanate of 1 in a gas chromatography measurement performed under the following GC conditions 1,
   when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
   when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
      - GC Conditions 1-
         Filler: DB-1 (film thickness) 1.5 µm
         Column: 0.53 mm in inner diameter × 60 m in length (manufactured by Agilent Technologies, Inc.)
         Oven temperature: increased from 130°C to 220°C at 3°C/min, and further increased to 300°C at 10°C/min after reaching 220°C
         Split ratio: pulsed splitless method
         Inlet temperature: 280°C
         Detector temperature: 300°C
         Carrier gas: N₂ 158 kPa, H₂ 55 kPa, Air 45 kPa (constant pressure control)
         Solvent: chloroform
         Sample concentration: 2.0%-by-mass chloroform solution
         Injection amount: 2 µL
         Detection method: FID
      - GC Conditions 2-
         Column: HP-50+, 0.25 mm in inner diameter × 30 m in length × 0.25 µm in film thickness (manufactured by Hewlett-Packard Company)
         Oven temperature: increased from 50°C to 280°C at 10°C/min, and held for 6 minutes after reaching 280°C
         Split ratio: pulsed splitless method
         Inlet temperature: 200°C
         Detector temperature: 280°C
         Carrier gas: He
         Carrier gas flow rate: 1.0 ml/min (constant flow rate control)
         Sample concentration: 1.0%-by-mass dichloromethane solution
         Injection amount: 1.0 µL
         Detection method: SIM (monitoring ion: m/z 180, 215) (content ratio of xylylene diisocyanate) 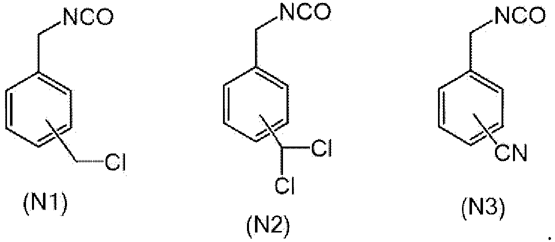
<20> A composition set used for the production of a polymerizable composition for an optical material, the composition set comprising:
   a polyiso(thio)cyanate composition comprising a polyiso(thio)cyanate compound; and
   the polythiol composition according to any one of <1> to <9>.
<21> The composition set according to <20>, wherein
   the polyiso(thio)cyanate composition comprises:
   xylylene diisocyanate; and
   at least one selected from the group consisting of the following compounds (N1), (N2), and (N3),
   when the polyiso(thio)cyanate composition comprises the compound (N1), the compound (N1) has a peak area of 0.20 ppm or more with respect to a peak area of xylylene diisocyanate of 1 in a gas chromatography measurement performed under the following GC conditions 1,
   when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
   when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
      - GC Conditions 1-
         Filler: DB-1 (film thickness) 1.5 µm
         Column: 0.53 mm in inner diameter × 60 m in length (manufactured by Agilent Technologies, Inc.)
         Oven temperature: increased from 130°C to 220°C at 3°C/min, and further increased to 300°C at 10°C/min after reaching 220°C
         Split ratio: pulsed splitless method
         Inlet temperature: 280°C
         Detector temperature: 300°C
         Carrier gas: N₂ 158 kPa, H₂ 55 kPa, Air 45 kPa (constant pressure control)
         Solvent: chloroform
         Sample concentration: 2.0%-by-mass chloroform solution
         Injection amount: 2 µL
         Detection method: FID
      - GC Conditions 2-
         Column: HP-50+, 0.25 mm in inner diameter × 30 m in length × 0.25 µm in film thickness (manufactured by Hewlett-Packard Company)
         Oven temperature: increased from 50°C to 280°C at 10°C/min, and held for 6 minutes after reaching 280°C
         Split ratio: pulsed splitless method
         Inlet temperature: 200°C
         Detector temperature: 280°C
         Carrier gas: He
         Carrier gas flow rate: 1.0 ml/min (constant flow rate control)
         Sample concentration: 1.0%-by-mass dichloromethane solution
         Injection amount: 1.0 µL
         Detection method: SIM (monitoring ion: m/z 180, 215) (content ratio of xylylene diisocyanate (XDI))

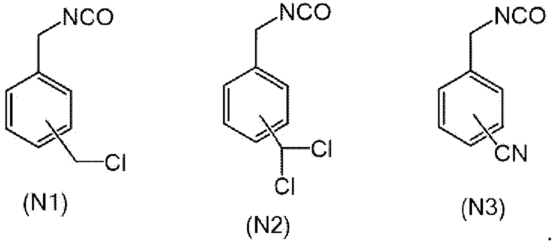

### Advantageous Effects of Invention

According to one aspect of the disclosure, a polythiol composition from which a thiourethane resin having excellent dyeability can be produced, and an application thereof is provided.

### DESCRIPTION OF EMBODIMENTS

In the disclosure, those numerical ranges that are expressed with "to" each denote a range that includes the numerical values stated before and after "to" as the lower limit value and the upper limit value, respectively.

In the disclosure, the term "step" encompasses not only a discrete step but also a step that cannot be clearly distinguished from other steps, as long as the intended purpose of the step is achieved.

In the disclosure, when there are plural substances that correspond to a component of a composition, the indicated amount of the component in the composition means, unless otherwise specified, a total amount of the plural substances existing in the composition.

In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.

### [Polythiol Composition]

The polythiol composition of the disclosure is a polythiol composition containing a polythiol compound,
in which the polythiol composition contains a compound C2 that has a retention time of from 16.0 minutes to 19.0 minutes in a high-performance liquid chromatography measurement performed under the following measurement conditions A (hereinafter, also simply referred to as "high-performance liquid chromatography measurement"), and
in the high-performance liquid chromatography measurement, the compound C2 has a peak area of 1.000 or more with respect to a total of 100 of a peak area of the compound C2 and a peak area of a main component of the polythiol composition.

### (Measurement Conditions A)

MIGHTYSIL (registered trademark) RP-18 GP manufactured by Kanto Chemical Co., Inc. (particle size S: 5 µm, column shape: Φ6 mm × 150 mm, product number: 25477-96) is used as a column,
a mixed solution of acetonitrile/0.01 mol/L-aqueous potassium dihydrogen phosphate solution = 60/40 (vol/vol) is used as a mobile phase,
a mixed solution of 160 mg of the polythiol composition and 10 mL of acetonitrile is used as a measurement solution,
a UV detector having a measurement wavelength of 230 nm is used as a detector,
a column temperature is set at 40°C,
a flow rate is set at 1.0 mL/min, and
a injection amount is set at 2 µL.

By using the polythiol composition of the disclosure as one of raw materials of a thiourethane resin, a thiourethane resin having excellent dyeability can be produced.

### (Polythiol Composition)

The term "polythiol composition" used herein means a composition containing at least one polythiol compound.

In the disclosure, a polythiol compound contained in the polythiol composition is also referred to as "polythiol component".

The polythiol composition may also contain, as an impurity, a component other than the polythiol compound.

It is preferred that the polythiol composition contains at least one polythiol compound as a main component.

It is noted here that "the polythiol composition contains at least one polythiol compound as a main component" means that a total content of the at least one polythiol compound is not less than 50% with respect to a total amount of the polythiol composition.

The total content of the at least one polythiol compound with respect to the total amount of the polythiol composition is preferably not less than 60%, more preferably not less than 70%, still more preferably not less than 80%.

Similarly, in the disclosure, a feature that a composition contains a certain component (hereinafter, referred to as "component X") "as a main component" means that the content of the component X (when the component X consists of two or more compounds, a total content of the two or more compounds) is not less than 50% with respect to a total amount of the composition.

The content of the component X as a main component with respect to the total amount of the composition is preferably not less than 60%, more preferably not less than 70%, still more preferably not less than 80%.

The "%" in the description of the above-described expression "contain ... as a main component" means a ratio (% by area) of a total area of all peaks of the component X (e.g., at least one polythiol compound) with respect to a total area of all peaks of the composition (e.g., a polythiol composition), which ratio is determined by high-performance liquid chromatography under the above-described measurement conditions A.

The polythiol composition is, for example, a polythiol composition containing a known polythiol compound.

The polythiol compound is not particularly limited as long as it is a compound containing two or more thiol groups (other name: mercapto groups).

With regard to the polythiol compound, reference can be made as appropriate to the aforementioned known documents (i.e., JP-A No. S63-46213, JP-A No. H2-270859, JP-A No. H7-252207, WO 2008/047626, and the like).

### (Compound C2)

The polythiol composition of the disclosure contains a compound C2.

The compound C2 is a compound that has a retention time of from 16.0 minutes to 19.0 minutes in a high-performance liquid chromatography measurement performed under the measurement conditions A.

In the polythiol composition of the disclosure, the compound C2 may be contained singly, or in combination of two or more kinds thereof.

The compound C2 may or may not be a polythiol compound; however, the compound C2 is preferably a polythiol compound.

The compound C2 is a component effective for improving the dyeability of a thiourethane resin produced using a polythiol composition.

In the polythiol composition of the disclosure, a peak area of the compound C2 with respect to a total of 100 of the peak area of the compound C2 and a peak area of the main component of the polythiol composition in the high-performance liquid chromatography measurement (hereinafter, also simply referred to as "peak area of the compound C2") is 1.000 or more, whereby an effect of improving the dyeability of the resulting thiourethane resin is exerted.

From the standpoint of further improving the dyeability of the resulting thiourethane resin, a lower limit of the peak area of the compound C2 is preferably 1.250, more preferably 1.500, still more preferably 1.600, yet still more preferably 2.500, further preferably 3.100, yet further preferably 4.000, further more preferably 7.000, yet further more preferably 8.000.

From the standpoint of reducing the yellowness (YI) of the resulting thiourethane resin, an upper limit of the peak area of the compound C2 is preferably 20.000, more preferably 15.000, still more preferably 13.000, yet still more preferably 9.000, further preferably 6.000.

The compound C2 has a molecular weight of, for example, 320.

The term "molecular weight" used herein refers to a value measured by high-performance liquid chromatography mass spectrometry under the following measurement conditions B.

### (Measurement Conditions B)

YMC-Pack (registered trademark) C18RS manufactured by YMC., Co., Ltd. (particle size S: 3 µm, column shape: Φ4.6 mm × 250 mm) is used as a column,
a mixed solution of 10 mM aqueous ammonium acetate solution/acetonitrile = 30/70 (vol/vol) is used as a mobile phase,
a UV detector having a measurement wavelength of 230 nm is used as a detector,
the column temperature is set at 40°C,
the flow rate is set at 0.9 mL/min, and
the following conditions are applied as mass spectrometry conditions:
   Ionization Mode: ESI+
   Capillary Voltage: 3.0 kV
   Cone Voltage: 20 V
   Extractor: 4V
   Source Temp: 120°C
   Desolvation Temp: 400°C
   Cone Gas Flow: 50 L/Hr
   Desolvation Gas Flow: 800 L/Hr
   Mass: m/z 50-800

The compound C2 is, for example, a polythiol compound having a molecular weight of 320.

Examples of the compound C2 that is a polythiol compound having a molecular weight of 320 include compounds represented by a molecular formula C₉H₂₀S₆.

Examples of the compound C2 that is a compound represented by a molecular formula C₉H₂₀S₆ include compounds represented by the following Formula (C2-1).

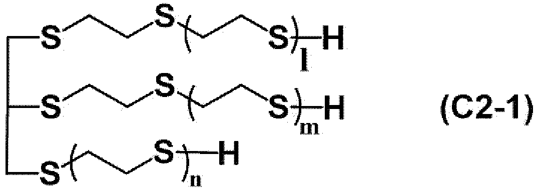

In Formula (C2-1), 1, m, and n each represent 0 or 1, and a total of l, m, and n is 1.

The compounds represented by Formula (C2-1) may each be a mixture of two or more kinds of compounds having different combinations of 1, m, and n.

When the compound C2 is a polythiol compound, the compound C2 preferably contains at least one selected from the group consisting of the following compounds (C2-1a), (C2-1b), and (C2-1c).

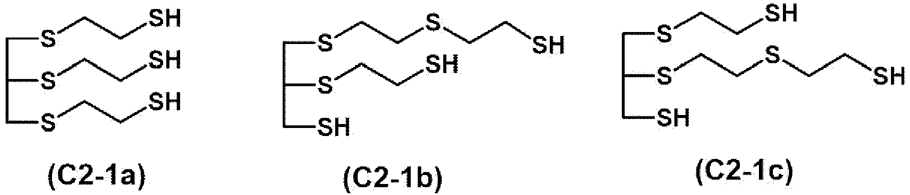

The compound (C2-1a) is a compound represented by Formula (C2-1) in which n is 1, and 1 and m are both 0,
the compound (C2-1b) is a compound represented by Formula (C2-1) in which 1 is 1, and n and m are both 0, and
the compound (C2-1b) is a compound represented by Formula (C2-1) in which m is 1, and 1 and m are both 0.

### (Compound C3)

The polythiol composition of the disclosure preferably further contains a compound C3.

By this, the dyeability of the resulting thiourethane resin is further improved.

The compound C3 is a compound that has a retention time of from 32.0 minutes to 35.0 minutes in the high-performance liquid chromatography measurement performed under the measurement conditions A.

When the polythiol composition of the disclosure contains the compound C3, the compound C3 may be contained singly, or in combination of two or more kinds thereof.

The compound C3 may or may not be a polythiol compound; however, the compound C3 is preferably a polythiol compound.

In the high-performance liquid chromatography measurement performed under the measurement conditions A, a peak area of the compound C3 with respect to a total of 100 of the peak area of the compound C3 and a peak area of the main component of the polythiol composition (hereinafter, also simply referred to as "peak area of the compound C3") is preferably 0.050 or more.

By this, the dyeability of the resulting thiourethane resin is further improved.

From the standpoint of further improving the dyeability of the resulting thiourethane resin, a lower limit of the peak area of the compound C3 is more preferably 0.100 or more, still more preferably 0.150 or more, yet still more preferably 0.600, further preferably 1.000, yet further preferably 1.500, further more preferably 2.000.

From the standpoint of reducing the yellowness (YI) of the resulting thiourethane resin, an upper limit of the peak area of the compound C3 is preferably 5.000, more preferably 4.000, still more preferably 3.500, yet still more preferably 2.500, further preferably 2.000, yet further preferably 1.500.

The compound C3 has a molecular weight of, for example, 426.

The term "molecular weight" used herein refers to a value measured by high-performance liquid chromatography mass spectrometry under the above-described measurement conditions B.

The compound C3 is, for example, a polythiol compound having a molecular weight of 426.

Examples of the compound C3 that is a polythiol compound having a molecular weight of 426 include compounds represented by a molecular formula C₁₂H₂₆S₈.

The compound C3 that is a compound represented by the molecular formula C₁₂H₂₆S₈ is, for example, at least one selected from the group consisting of compounds represented by the following Formula (C3-1), (C3-2), or (C3-3).

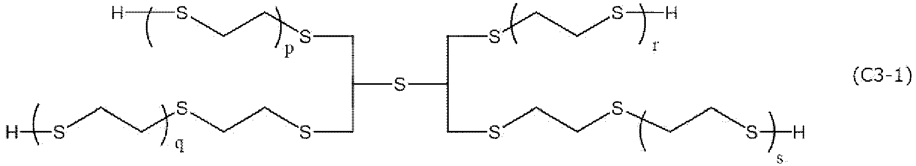

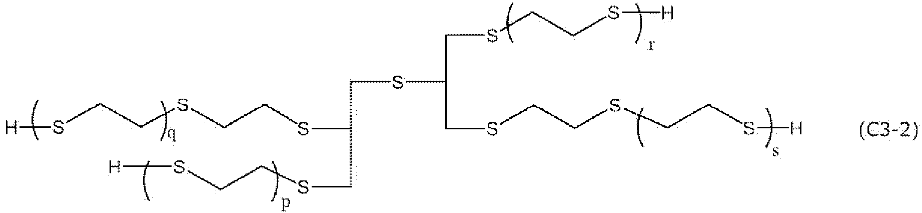

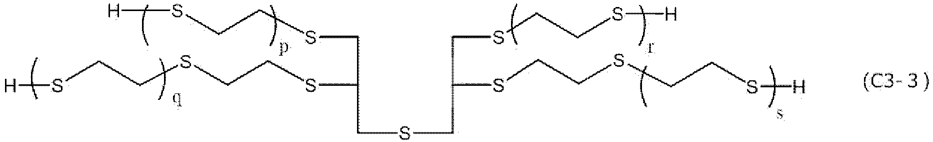

In Formulae (C3-1) to (3-3), p, q, r, and s each represent 0 or 1, and a total of p, q, r, and s is 1.

### (Compound C4)

The polythiol composition of the disclosure preferably further contains a compound C4.

By this, the dyeability of the resulting thiourethane resin is further improved.

The compound C4 is a compound that has a retention time of from 21.5 minutes to 25.0 minutes in the high-performance liquid chromatography measurement performed under the measurement conditions A.

When the polythiol composition of the disclosure contains the compound C4, the compound C4 may be contained singly, or in combination of two or more kinds thereof.

The compound C4 may or may not be a polythiol compound; however, the compound C4 is preferably a polythiol compound.

In the high-performance liquid chromatography measurement performed under the measurement conditions A, a peak area of the compound C4 with respect to a total of 100 of the peak area of the compound C4 and a peak area of the main component of the polythiol composition (hereinafter, also simply referred to as "peak area of the compound C4") is preferably 0.030 or more.

By this, the dyeability of the resulting thiourethane resin is further improved.

From the standpoint of further improving the dyeability of the resulting thiourethane resin, a lower limit of the peak area of the compound C4 is preferably 0.040, more preferably 0.500, still more preferably 0.600.

From the standpoint of reducing the yellowness (YI) of the resulting thiourethane resin, an upper limit of the peak area of the compound C4 is preferably 1.300, more preferably 1.200, still more preferably 1.100.

The compound C4 has a molecular weight of, for example, 380.

The term "molecular weight" used herein refers to a value measured by high-performance liquid chromatography mass spectrometry under the above-described measurement conditions B.

The compound C4 is, for example, a polythiol compound having a molecular weight of 380.

Examples of the compound C4 that is a polythiol compound having a molecular weight of 380 include compounds represented by a molecular formula C₁₁H₂₄S₇.

The compound C4 that is a compound represented by the molecular formula C₁₁H₂₄S₇ is, for example, at least one selected from the group consisting of compounds represented by the following Formula (C4-1).

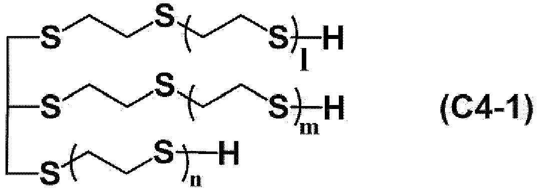

In Formula (C4-1), 1, m, and n each represent 0, 1, or 2, and a total of l, m, and n is 2.

The compounds represented by Formula (C4-1) may each be a mixture of two or more kinds of compounds having different combinations of 1, m, and n.

### (Polythiol Compound (XA))

The main component in the polythiol composition of the disclosure is preferably a polythiol compound, more preferably a polythiol compound (XA) containing three or more mercapto groups.

The polythiol compound (XA) is a polythiol compound containing three or more mercapto groups. However, when the above-described compound C2 is a "polythiol compound containing three or more mercapto groups", the compound C2 is not included in the scope of the polythiol compound (XA). Similarly, when the polythiol composition of the disclosure contains the above-described compound C3 that is a "polythiol compound containing three or more mercapto groups", the compound C3 is not included in the scope of the polythiol compound (XA).

The polythiol compound (XA) is preferably at least one selected from the group consisting of:
4-mercaptomethyl-1,8-dimercapto-3 ,6-dithiaoctane,
4,8-dimercaptomethyl-1,11-dimercapto-3,6,9-trithiaundecane,
4,7-dimercaptomethyl-1,11-dimercapto-3,6,9-trithiaundecane, and
5,7-dimercaptomethyl-1,11-dimercapto-3,6,9-trithiaundecane.

The polythiol compound (XA) is preferably a polythiol component A1 that is 4-mercaptomethyl-1,8-dimercapto-3,6-dithiaoctane, or a polythiol component A2 that is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

The polythiol compound (XA) is more preferably the polythiol component A1.

The polythiol component A1 (i.e., 4-mercaptomethyl-1,8-dimercapto-3,6-dithiaoctane) is the following compound (A1).

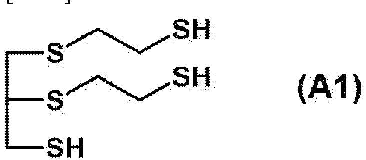

Under the above-described measurement conditions A, the polythiol component A1 has a retention time of from 12.0 minutes to 13.0 minutes, and the polythiol component A2 has a retention time of from 22.0 minutes to 26.0 minutes.

### (Other Components)

The polythiol composition of the disclosure may also contain at least one other component (e.g., other polythiol compound, a component other than a polythiol compound) in addition to the main component.

Examples of the other polythiol compound include methanedithiol, 1,2-ethanedithiol, 1,2,3-propanetrithiol, pentaerythritol tetrakis(2-mercaptoacetate), pentaerythritol tetrakis(3-mercaptopropionate), bis(mercaptoethyl) sulfide, 2,5-dimercaptomethyl-1,4-dithiane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

### [Polymerizable Composition for Optical Material]

The polymerizable composition for an optical material according to the disclosure (hereinafter, also simply referred to as "polymerizable composition") contains:
a polyiso(thio)cyanate compound; and
the above-described polythiol composition of the disclosure.

The polymerizable composition of the disclosure contains the polythiol composition of the disclosure and, therefore, exerts the same effects as those of the polythiol composition of the disclosure.

### (Polyiso(thio)cyanate Compound)

The polyiso(thio)cyanate compound is not particularly limited as long as it is a compound having at least two iso(thio)cyanate groups in one molecule.

Specific examples of the polyiso(thio)cyanate compound include:
aliphatic polyisocyanate compounds, such as tetramethylene diisocyanate, pentamethylene diisocyanate, hexamethylene diisocyanate, heptamethylene diisocyanate, octamethylene diisocyanate, 2,2,4-trimethylhexamethylene diisocyanate, 2,4,4-trimethylhexamethylene diisocyanate, lysine diisocyanate methyl ester, lysine triisocyanate, and xylylene diisocyanate;
alicyclic polyisocyanate compounds, such as isophorone diisocyanate, bis(isocyanatomethyl)cyclohexane, bis(isocyanatocyclohexyl)methane, dicyclohexyldimethylmethane diisocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds, such as tolylene diisocyanate, 4,4'-diphenylmethane diisocyanate, diphenyl sulfide-4,4'-diisocyanate, and phenylene diisocyanate;
heterocyclic polyisocyanate compounds, such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;
aliphatic polyisothiocyanate compounds, such as hexamethylene diisothiocyanate, lysine diisothiocyanate methyl ester, lysine triisothiocyanate, and xylylene diisothiocyanate;
alicyclic polyisothiocyanate compounds, such as isophorone diisothiocyanate, bis(isothiocyanatomethyl)cyclohexane, bis(isothiocyanatocyclohexyl)methane, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane;
aromatic polyisothiocyanate compounds, such as tolylene diisothiocyanate, 4,4'-diphenylmethane diisothiocyanate, and diphenyl disulfide-4,4'-diisothiocyanate; and
sulfur-containing heterocyclic polyisothiocyanate compounds, such as 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane.

The polyiso(thio)cyanate compound may contain at least one selected from the above-exemplified compounds.

As the polyiso(thio)cyanate compound, for example, a halogen substitute such as a bromine substitute or a chlorine substitute, an alkyl substitute, an alkoxy substitute, a nitro substitute, a prepolymer-type product modified with a polyhydric alcohol, a carbodiimide-modified product, a urea-modified product, a burette-modified product, or a dimerization or trimerization reaction product of any of the above-exemplified compounds can be used as well.

The polyiso(thio)cyanate compound preferably contains a polyisocyanate compound, more preferably contains at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

A mixing ratio of the polythiol composition and the polyiso(thio)cyanate compound is not particularly limited and, for example, a molar ratio between the mercapto groups of the polythiol compound contained in the polythiol composition and the iso(thio)cyanate groups of the polyiso(thio)cyanate compound (mercapto groups/iso(thio)cyanate groups) is preferably from 0.5 to 3.0, more preferably from 0.6 to 2.0, still more preferably from 0.8 to 1.3. When the mixing ratio is in the above-described range, various performance required for a plastic lens or the like, such as refractive index and heat resistance, tend to be satisfied in a well-balanced manner.

The polymerizable composition of the disclosure may also contain a polyiso(thio)cyanate composition containing a polyiso(thio)cyanate compound.

In other words, the polymerizable composition of the disclosure may contain:
a polyiso(thio)cyanate composition containing a polyiso(thio)cyanate compound; and
the above-described polythiol composition of the disclosure.

When the polymerizable composition of the disclosure contains a polyiso(thio)cyanate composition, the contained polyiso(thio)cyanate composition may contain a polyiso(thio)cyanate compound singly, or in combination of two or more kinds thereof.

When the polymerizable composition of the disclosure contains a polyiso(thio)cyanate composition, the polyiso(thio)cyanate composition preferably contains xylylene diisocyanate.

By this, the polyiso(thio)cyanate composition exhibits superior stability, and a resin formed using the polyiso(thio)cyanate composition attains superior transparency (i.e., development of turbidity and/or yellowing is/are further inhibited).

Hereinafter, a polyiso(thio)cyanate composition containing xylylene diisocyanate is also referred to as "XDI composition".

The XDI composition preferably contains xylylene diisocyanate as a main component.

The XDI composition preferably contains at least one selected from the group consisting of the following compounds (N1), (N2), and (N3).

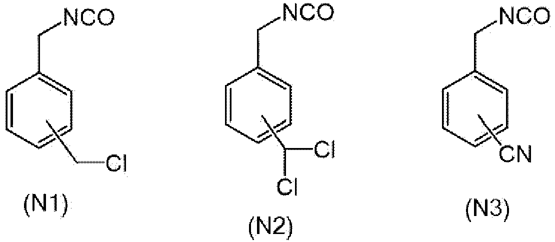

Aspects of the XDI composition that are preferred from the standpoint of obtaining superior stability of the polyiso(thio)cyanate composition and superior transparency of a resin formed using the polyiso(thio)cyanate composition will now be described.

When the XDI composition contains the compound (N1), the compound (N1) preferably has a peak area of 0.20 ppm or more with respect to a peak area of xylylene diisocyanate of 1 in a gas chromatography measurement performed under the following GC conditions 1:
-GC Conditions 1-
Filler: DB-1 (film thickness) 1.5 µm
Column: 0.53 mm in inner diameter × 60 m in length (manufactured by Agilent Technologies, Inc.)
Oven temperature: increased from 130°C to 220°C at 3°C/min, and further increased to 300°C at 10°C/min after reaching 220°C
Split ratio: pulsed splitless method
Inlet temperature: 280°C
Detector temperature: 300°C
Carrier gas: N₂ 158 kPa, H₂ 55 kPa, Air 45 kPa (constant pressure control)
Solvent: chloroform
Sample concentration: 2.0%-by-mass chloroform solution
Injection amount: 2 µL
Detection method: FID.

The peak area of the compound (N1) is more preferably 5.0 ppm or more, still more preferably 50 ppm or more, yet still more preferably 100 ppm or more, with respect to a peak area of xylylene diisocyanate of 1.

The peak area of the compound (N1) is preferably 4,000 ppm or less, more preferably 3,000 ppm or less, still more preferably 2,000 ppm or less, yet still more preferably 1,500 ppm or less, further preferably 1,000 ppm or less, with respect to a peak area of xylylene diisocyanate of 1.

The peak area of the compound (N1) can be measured in accordance with the method described in the paragraph [0377] of Japanese Patent No. 6373536.

When the XDI composition contains the compound (N2), the compound (N2) preferably has a peak area of 0.05 ppm or more with respect to a peak area of xylylene diisocyanate of 1 in a gas chromatography measurement performed under the following GC conditions 2:
-GC Conditions 2-
Column: HP-50+, 0.25 mm in inner diameter × 30 m in length × 0.25 µm in film thickness (manufactured by Hewlett-Packard Company)
Oven temperature: increased from 50°C to 280°C at 10°C/min, and held for 6 minutes after reaching 280°C
Split ratio: pulsed splitless method
Inlet temperature: 200°C
Detector temperature: 280°C
Carrier gas: He
Carrier gas flow rate: 1.0 ml/min (constant flow rate control)
Sample concentration: 1.0%-by-mass dichloromethane solution
Injection amount: 1.0 µL
Detection method: SIM (monitoring ion: m/z 180, 215) (content ratio of xylylene diisocyanate (XDI)).

The peak area of the compound (N2) is more preferably 0.1 ppm or more, still more preferably 0.3 ppm or more, yet still more preferably 0.6 ppm or more, with respect to a peak area of xylylene diisocyanate of 1.

The peak area of the compound (N2) is preferably 200 ppm or less, more preferably 150 ppm or less, still more preferably 100 ppm or less, yet still more preferably 80 ppm or less, further preferably 70 ppm or less, yet further preferably 60 ppm or less, with respect to a peak area of xylylene diisocyanate of 1.

The peak area of the compound (N2) can be measured in accordance with the method described in the paragraphs [0375] and [0376] of Japanese Patent No. 6373536.

When the XDI composition contains the compound (N3), the compound (N3) preferably has a peak area of 0.10 ppm or more with respect to a peak area of xylylene diisocyanate of 1 in a gas chromatography measurement performed under the above-described GC conditions 1:
The peak area of the compound (N3) is more preferably 0.1 ppm or more, still more preferably 3.0 ppm or more, yet still more preferably 5.0 ppm or more, with respect to a peak area of xylylene diisocyanate of 1.

The peak area of the compound (N3) is preferably 1,000 ppm or less, more preferably 500 ppm or less, still more preferably 300 ppm or less, yet still more preferably 100 ppm or less, further preferably 75 ppm or less, with respect to a peak area of xylylene diisocyanate of 1.

The peak area of the compound (N3) can be measured in accordance with the method described in the paragraph [0377] of Japanese Patent No 6373536.

The XDI composition has an acid content of preferably 3,000 ppm or less, more preferably 2,000 ppm or less, still more preferably 1,000 ppm or less, yet still more preferably 100 ppm or less, further preferably 50 ppm or less, yet further preferably 30 ppm or less, further more preferably less than 15 ppm.

A lower limit value of the acid content of the XDI composition is not particularly limited; however, it is, for example, 1 ppm.

The acid content of the XDI composition can be measured in accordance with the method described in the paragraph [0091] of WO 2021/256417.

The XDI composition may also contain a stabilizer.

The polymerizable composition of the disclosure may also contain other components in addition to the polythiol composition and the polyiso(thio)cyanate compound.

Examples of the other components include a polymerization catalyst, an internal mold release agent, a resin modifier, a chain extender, a crosslinking agent, a radical scavenger, a light stabilizer, a UV absorber, an antioxidant, an oil-soluble dye, a filler, an adhesion improver, an antimicrobial agent, an antistatic agent, a dye, a fluorescent brightener, a fluorescent pigment, and a blue ink agent such as an inorganic pigment.

Examples of the polymerization catalyst include tertiary amine compounds, inorganic and organic acid salts thereof, metal compounds, quaternary ammonium salts, and organic sulfonic acids.

As the internal mold release agent, an acidic phosphoric acid ester can be used. Examples of the acidic phosphoric acid ester include phosphoric acid monoesters and phosphoric acid diesters, and these may be used singly, or in combination of two or more kinds thereof.

Examples of the resin modifier include episulfide compounds, alcohol compounds, amine compounds, epoxy compounds, organic acids and anhydrides thereof, and olefin compounds containing a (meth)acrylate compound or the like.

The polymerizable composition of the disclosure can be obtained by mixing the above-described components.

A method of producing the polymerizable composition of the disclosure is not particularly limited, and examples thereof include the following production method according to one embodiment.

For the production of the polymerizable composition of the disclosure, the below-described composition set according to one embodiment may be used.

### [Method of Producing Polymerizable Composition for Optical Material]

The method of producing a polymerizable composition for an optical material according to one embodiment of the disclosure includes the step of mixing a polyiso(thio)cyanate composition containing a polyiso(thio)cyanate compound with the above-described polythiol composition of the disclosure.

According to the method of producing a polymerizable composition for an optical material according to one embodiment of the disclosure, the above-described polymerizable composition of the disclosure can be easily produced.

In the method of producing a polymerizable composition for an optical material according to one embodiment of the disclosure, the polyiso(thio)cyanate composition preferably contains:
xylylene diisocyanate; and
at least one selected from the group consisting of the above-described compounds (N1), (N2), and (N3).

In this case, it is more preferred that:
when the polyiso(thio)cyanate composition contains the compound (N1), the compound (N1) has a peak area of 0.20 ppm or more with respect to a peak area of xylylene diisocyanate of 1 in a gas chromatography measurement performed under the above-described GC conditions 1;
when the polyiso(thio)cyanate composition contains the compound (N2), the compound (N2) has a peak area of 0.05 ppm or more with respect to a peak area of xylylene diisocyanate of 1 in a gas chromatography measurement performed under the above-described GC conditions 2; and
when the polyiso(thio)cyanate composition contains the compound (N3), the compound (N3) has a peak area of 0.10 ppm or more with respect to a peak area of xylylene diisocyanate of 1 in a gas chromatography measurement performed under the above-described GC conditions 1.

The method of producing a polymerizable composition for an optical material according to one embodiment of the disclosure may also include other steps in addition to the mixing step if necessary.

Examples of the other steps include the step of mixing a mixture obtained by the mixing step with the above-described components other than the polythiol composition and the polyiso(thio)cyanate compound that are contained in the polymerizable composition of the disclosure.

Alternatively, these other components may be mixed with the polyiso(thio)cyanate composition and xylylene diisocyanate in the mixing step.

### [Composition Set]

The composition set according to one embodiment of the disclosure is a composition set used for the production of a polymerizable composition for an optical material, and includes:
a polyiso(thio)cyanate composition containing a polyiso(thio)cyanate compound; and
the above-described polythiol composition of the disclosure.

With the composition set according to one embodiment, the above-described polymerizable composition of the disclosure can be easily produced.

For example, using the composition set according to one embodiment, the above-described mixing step in the method of producing a polymerizable composition for an optical material according to one embodiment can be carried out.

In the composition set according to one embodiment, the polyiso(thio)cyanate composition preferably contains:
xylylene diisocyanate; and
at least one selected from the group consisting of the above-described compounds (N1), (N2), and (N3).

In this case, it is more preferred that:
when the polyiso(thio)cyanate composition contains the compound (N1), the compound (N1) has a peak area of 0.20 ppm or more with respect to a peak area of xylylene diisocyanate of 1 in a gas chromatography measurement performed under the above-described GC conditions 1;
when the polyiso(thio)cyanate composition contains the compound (N2), the compound (N2) has a peak area of 0.05 ppm or more with respect to a peak area of xylylene diisocyanate of 1 in a gas chromatography measurement performed under the above-described GC conditions 2; and
when the polyiso(thio)cyanate composition contains the compound (N3), the compound (N3) has a peak area of 0.10 ppm or more with respect to a peak area of xylylene diisocyanate of 1 in a gas chromatography measurement performed under the above-described GC conditions 1.

### [Resin]

The resin of the disclosure is a cured product of the above-described polymerizable composition of the disclosure.

The resin of the disclosure is obtained by curing the above-described polymerizable composition of the disclosure.

The polymerizable composition can be cured by polymerizing the monomers contained in the polymerizable composition (specifically the polythiol composition and the polyiso(thio)cyanate compound; the same applies hereinafter). As a pretreatment of this polymerization, the polymerizable composition may be subjected to filtration, degassing, and the like.

The conditions for the polymerization of the monomers contained in the polymerizable composition (e.g., polymerization temperature and polymerization time) are set as appropriate, taking into consideration the formulation of the composition, the types and the amounts of the monomers used in the composition, the type and the amount of a polymerization catalyst used in the composition, and the properties of the below-described mold if used, and the like.

The polymerization temperature is, for example, from -50°C to 150°C, or from 10°C to 150°C.

The polymerization time is, for example, from 1 hour to 200 hours, or from 1 hour to 80 hours.

The resin of the disclosure may also be obtained by performing a treatment such as annealing on a polymer obtained by the polymerization of the monomers.

The temperature of this annealing is, for example, from 50°C to 150°C, from 90°C to 140°C, or from 100°C to 130°C.

### [Formed Body]

The formed body of the disclosure contains the resin of the disclosure.

The formed body of the disclosure is obtained by curing the polymerizable composition of the disclosure in the same manner as the resin of the disclosure.

Preferred conditions for the curing of the polymerizable composition, i.e., the polymerization of the monomers contained in the polymerizable composition, are as described above.

One example of a preferred method of producing the formed body of the disclosure is cast polymerization.

In the cast polymerization, first, the above-described polymerizable composition is injected between casting molds held by a gasket, a tape, or the like. In this process, if necessary, a defoaming treatment, a filtration treatment, and the like may be performed.

Next, the monomers contained in the polymerizable composition injected between the casting molds are polymerized to cure the composition between the casting molds and thereby obtain a cured product. Subsequently, the cured product is removed from the casting molds to obtain a resin-containing formed body.

The polymerization of the monomers may also be performed by heating the polymerizable composition. This heating can be performed using, for example, a heating apparatus equipped with a mechanism for heating a material to be heated in an oven, water, or the like.

In the cast polymerization, formed bodies of various shapes (e.g., the below-described optical material) can be obtained by modifying the shape of the casting molds.

### [Optical Material]

The optical material of the disclosure contains the resin of the disclosure.

The optical material of the disclosure is obtained by curing the polymerizable composition of the disclosure in the same manner as the resin of the disclosure.

Preferred conditions for the curing of the polymerizable composition, i.e., the polymerization of the monomers contained in the polymerizable composition, are as described above.

Examples of the optical material include lenses (e.g., eyeglass lenses, camera lenses, and polarizing lenses), and light-emitting diodes (LEDs).

The optical material of the disclosure may include a coating layer formed on one side or both sides of the resin of the disclosure (or a formed body containing the resin of the disclosure).

Specific examples of the coating layer include a primer layer, a hard coat layer, an anti-reflection layer, an anti-fog coating layer, an anti-fouling layer, and a water-repellent layer.

These coating layers may each be formed singly, or plural coating layers may be formed in the form of a multilayer structure. When coating layers are formed on both sides, the same coating layer may be formed on the respective sides, or different coating layers may be formed on the respective sides.

Components of the coating layer can be selected as appropriate in accordance with the intended purpose.

Examples of the components of the coating layer include a resin (e.g., a urethane resin, an epoxy resin, a polyester resin, a melamine resin, or a polyvinyl acetal resin), an infrared absorber, a light stabilizer, an antioxidant, a photochromic compound, a dye, a pigment, and an antistatic agent.

With regard to the eyeglass lens and the coating layer, reference can be made as appropriate to the descriptions of known documents, such as WO 2017/047745.

### EXAMPLES

Examples of the disclosure will now be described; however, the disclosure is not limited to the below-described Examples.

### <Production of Composition (A1-1) Containing Polythiol Component A1 as Main Component>

The production of a composition (A1-1) containing a polythiol component A1 as a main component was carried out. The details thereof are described below.

Into a reactor, 124.6 parts by mass of 2-mercaptoethanol and 18.3 parts by mass of deaerated water were charged. To this reactor, 101.5 parts by mass of a 32%-by-mass aqueous sodium hydroxide solution was added dropwise at a temperature of from 12°C to 35°C over a period of 40 minutes, after which 73.6 parts by mass of epichlorohydrin was added dropwise at a temperature of from 29°C to 36°C over a period of 4.5 hours, and this was followed by 40-minute stirring. As a result, 1,3-bis(2-hydroxyethylthio)-2-propanol was generated in the reactor. This generation was confirmed by NMR data.

Next, into the reactor, 331.5 parts by mass of 35.5%-by-mass hydrochloric acid was charged, 183.8 parts by mass of thiourea having a purity of 99.90% by mass was subsequently charged, and the resultant was stirred under reflux at 110°C for 3 hours to perform a thiuronium chlorination reaction. Then, after cooling the resulting liquid in the reactor to 45°C, 320.5 parts by mass of toluene was added thereto, and the thus obtained liquid was cooled to 31°C, and 243.1 parts by mass of a 25%-by-mass aqueous ammonia solution was subsequently charged thereto at a temperature of 31°C to 41°C over a period of 44 minutes, after which the resultant was stirred at a temperature of from 54°C to 62°C for 3 hours to perform a hydrolysis reaction. As a result, a toluene solution of a polythiol composition containing a polythiol component A1 (i.e., 4-mercaptomethyl-1,8-dimercapto-3,6-dithiaoctane) as a main component was obtained. This toluene solution was acid-washed with 162.8 parts by mass of 35.5%-by-mass hydrochloric acid at a temperature of from 35°C to 43°C for 1 hour. An operation of washing the thus acid-washed toluene solution with 174.1 parts by mass of deaerated water at a temperature of from 35°C to 45°C for 30 minutes was performed twice. The toluene solution after the second washing with deaerated water was washed with 162.1 parts by mass of 0.1%-by-mass aqueous ammonia for 30 minutes. After this washing with aqueous ammonia, an operation of washing the toluene solution with 174.2 parts by mass of deaerated water at a temperature of 35°C to 45°C for 30 minutes was performed twice. Thereafter, toluene and a trace amount of water were removed from the toluene solution under reduced pressure with heating, and the resultant was treated by silica gel column chromatography and then vacuum-filtered through a 1.2-µm PTFE-type membrane filter, whereby a composition (A1-1) (205.0 parts by mass) containing the polythiol component A1 as a main component was obtained.

### <Production of Composition (A1-2) Containing Polythiol Component A1 as Main Component>

The production of a composition (A1-2) containing the polythiol component A1 as a main component was carried out.

In the production of the composition (A1-2), as compared to the production of the composition (A1-1), the residual amount of 2-mercaptoethanol was increased by reducing the amount of sodium hydroxide used in the stage of generating 1,3-bis(2-hydroxyethylthio)-2-propanol. By this, the production of the composition (A1-2) was carried out under a condition where, as compared to the production of the composition (A1-1), a compound C2 (i.e., at least one selected from the group consisting of the following compounds (C2-1a), (C2-1b), and (C2-1c)), which is a reaction by-product with respect to the polythiol component A1 (i.e., the following compound (A1)) that is a reaction main product, was more likely to be generated.

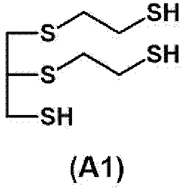

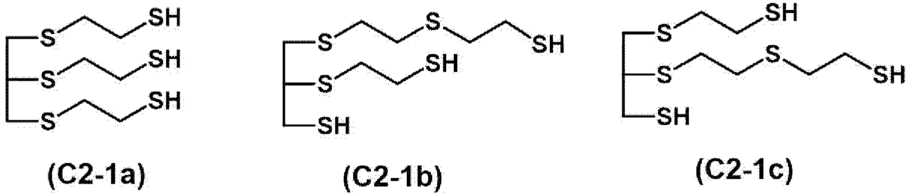

The details of the production of the composition (A1-2) are described below.

Into a reactor, 124.8 parts by mass of 2-mercaptoethanol and 22.5 parts by mass of deaerated water were charged. To this reactor, 99.0 parts by mass of a 30.8%-by-mass aqueous sodium hydroxide solution was added dropwise at a temperature of from 12°C to 35°C over a period of 40 minutes, after which 74.0 parts by mass of epichlorohydrin was added dropwise at a temperature of from 29°C to 36°C over a period of 4.5 hours, and this was followed by 40-minute stirring. As a result, 1,3-bis(2-hydroxyethylthio)-2-propanol was generated in the reactor. This generation was confirmed by NMR data.

Next, into the reactor, 331.7 parts by mass of 35.5%-by-mass hydrochloric acid was charged, 182.7 parts by mass of thiourea having a purity of 99.90% by mass was subsequently charged, and the resultant was stirred under reflux at 110°C for 3 hours to perform a thiuronium chlorination reaction. Then, after cooling the resulting liquid in the reactor to 45°C, 355.0 parts by mass of toluene was added thereto, and the thus obtained liquid was cooled to 31°C, and 245.2 parts by mass of a 25%-by-mass aqueous ammonia solution was subsequently charged thereto at a temperature of 31°C to 41°C over a period of 44 minutes, after which the resultant was stirred at a temperature of from 54°C to 62°C for 3 hours to perform a hydrolysis reaction. As a result, a toluene solution of a polythiol composition containing the polythiol component A1 (i.e., 4-mercaptomethyl-1,8-dimercapto-3,6-dithiaoctane) as a main component was obtained. This toluene solution was acid-washed with 147.8 parts by mass of 35.5%-by-mass hydrochloric acid at a temperature of from 35°C to 43°C for 1 hour. An operation of washing the thus acid-washed toluene solution with 147.8 parts by mass of deaerated water at a temperature of from 35°C to 45°C for 30 minutes was performed twice. The toluene solution after the second washing with deaerated water was washed with 147.8 parts by mass of 0.1%-by-mass aqueous ammonia for 30 minutes. After this washing with aqueous ammonia, an operation of washing the toluene solution with 147.8 parts by mass of deaerated water at a temperature of 35°C to 45°C for 30 minutes was performed twice. Thereafter, toluene and a trace amount of water were removed from the toluene solution under reduced pressure with heating, and the resultant was then vacuum-filtered through a 3.0-µm PTFE-type membrane filter, whereby a composition (A1-2) (199.7 parts by mass) containing the polythiol component A1 as a main component was obtained.

### [Examples 1 to 6]

### <Production of Polythiol Compositions>

The above-obtained composition (A1-1) and the above-obtained composition (A1-2) were mixed at a mixing mass ratio [composition (A1-1)/composition (A1-2)] of 99/1 to obtain a polythiol composition of Example 1 which contained the polythiol component A1 as a main component along with a compound C2 and a compound C3 as reaction by-products.

A polythiol composition of Example 2 was obtained in the same manner as the polythiol composition of Example 1, except that the mixing mass ratio [composition (A1-1)/composition (A1-2)] was changed to 75/25.

A polythiol composition of Example 3 was obtained in the same manner as the polythiol composition of Example 1, except that the mixing mass ratio [composition (A1-1)/composition (A1-2)] was changed to 60/40.

A polythiol composition of Example 4 was obtained in the same manner as the polythiol composition of Example 1, except that the mixing mass ratio [composition (A1-1)/composition (A1-2)] was changed to 40/60.

A polythiol composition of Example 5 was obtained in the same manner as the polythiol composition of Example 1, except that the mixing mass ratio [composition (A1-1)/composition (A1-2)] was changed to 20/80.

A polythiol composition of Example 6 was obtained in the same manner as the polythiol composition of Example 1, except that the mixing mass ratio [composition (A1-1)/composition (A1-2)] was changed to 0/100.

### <Measurement of Peak Areas of Compounds C2, C3, and C4>

For each of the thus obtained polythiol compositions of Examples 1 to 5, the peak area of the compound C2, the peak area of the compound C3, and the peak area of the compound C4 were each determined by a high-performance liquid chromatography measurement under the above-described measurement conditions A.

It is noted here that:
the compound C2 is a compound that has a retention time of from 16.0 minutes to 19.0 minutes in the high-performance liquid chromatography measurement performed under the measurement conditions A;
the compound C3 is a compound that has a retention time of from 32.0 minutes to 35.0 minutes in the high-performance liquid chromatography measurement performed under the measurement conditions A;
the compound C4 is a compound that has a retention time of from 21.5 minutes to 25.0 minutes in the high-performance liquid chromatography measurement performed under the measurement conditions A;
the peak area of the compound C2 is a peak area with respect to a total peak area of 1 of the peak area of the compound C2 and the peak area of the main component (i.e., polythiol component A1) of each polythiol composition;
the peak area of the compound C3 is a peak area with respect to a total peak area of 1 of the peak area of the compound C3 and the peak area of the main component (i.e., polythiol component A1) of each polythiol composition; and
the peak area of the compound C4 is a peak area with respect to a total peak area of 1 of the peak area of the compound C4 and the peak area of the main component (i.e., polythiol component A1) of each polythiol composition.

The results are shown in Table 1.

### <Production of Polymerizable Composition and Resin Formed Body>

The following materials were mixed and dissolved at 20°C:
an XDI composition X1 (52 parts by mass) as the above-described XDI composition that is one aspect of a polyiso(thio)cyanate composition;
dibutyl tin dichloride (0.01 parts by mass) as a curing catalyst;
ZELEC UN (trade name, manufactured by Stepan Company; acidic phosphate) (0.10 parts by mass) as a mold release agent; and
BIOSORB 583 (manufactured by Kyodo Chemical Co., Ltd.; UV absorber) (1.5 parts by mass) as a UV absorber.

The thus obtained mixture was mixed with 48 parts by mass of any one of the polythiol compositions of Examples 1 to 5 to obtain a polymerizable composition as a mixed uniform liquid.

Next, the thus obtained polymerizable composition was defoamed at 600 Pa for 1 hour.

The thus defoamed polymerizable composition was filtered through a 1-µm TEFLON (registered trademark) filter, and the resulting filtrate was injected between a pair of glass molds immobilized by a tape, and then, this pair of glass molds was placed in an oven, after which the oven internal temperature was set at 10°C. Subsequently, the oven internal temperature was raised from 10°C to 120°C over a period of 38 hours. By this process, monomers (polyisocyanate compound and polythiol composition) contained in the polymerizable composition were polymerized to form a resin-containing formed body (i.e., a cured product of the polymerizable composition) between the pair of glass molds.

Thereafter, the inside of the oven was cooled, and the pair of glass molds was subsequently taken out of the oven, after which the resin-containing formed body (hereinafter, referred to as "resin formed body") was removed from the pair of glass molds.

In the above-described manner, a resin formed body in the form of a 9 mm-thick flat plate was obtained.

As a result of performing a gas chromatography measurement of the XDI composition X1 under each of the above-described GC conditions 1 and 2, it was found that:
the compound (N1) had a peak area of 0.20 ppm or more (specifically, 600 ppm) with respect to a peak area of xylylene diisocyanate of 1;
the compound (N2) had a peak area of 0.05 ppm or more (specifically, 18 ppm) with respect to a peak area of xylylene diisocyanate of 1; and
the compound (N3) had a peak area of 0.10 ppm or more (specifically, 100 ppm) with respect to a peak area of xylylene diisocyanate of 1.

### <Measurement and Evaluation of Resin Formed Bodies>

For the thus obtained resin formed body in the form of a 9 mm-thick flat plate, the yellowness (YI) was measured, and the dyeability was evaluated.

The results thereof are shown in Table 1.

### (Yellowness (YI))

The yellowness (YI) of the resin formed body in the form of a 9 mm-thick flat plate was measured using a spectrophotometer CM-5 manufactured by Konica Minolta, Inc.

The lower the yellowness (YI) of the resin formed body, the superior is the hue of the resin formed body.

### (Dyeability)

The resin formed body in the form of a 9 mm-thick flat plate was dyed, and the light transmittance of the thus dyed resin formed body was measured to evaluate the dyeability of the resin formed body.

The lower the light transmittance of the dyed resin formed body, the superior is the dyeability of the resin formed body.

The details are described below.

To 1,985.5 g of pure water, 1.5 g of "FSP Red E-A" (manufactured by Futaba Sangyo Co., Ltd., dye), 1.0 g of "FSP Yellow P-E" (manufactured by Futaba Sangyo Co., Ltd., dye), 4.0 g of "FSP Blue AUL-S" (manufactured by Futaba Sangyo Co., Ltd., dye), 4.0 g of "NICCA SUNSOLT #7000" (manufactured by NICCA Chemical Co., Ltd., dye dispersant), and 4.0 g of "DK-CN" (manufactured by Daiwa Chemical Industries Co., Ltd., dyeing aid) were added, and these materials were mixed to obtain a dye dispersion.

The resin formed body in the form of a 9 mm-thick flat plate was immersed in the thus obtained dye dispersion at 90°C for 30 minutes and thereby dyed.

The light transmittance (%) of the thus dyed resin formed body at a wavelength of from 350 nm to 800 nm was measured and, from the measurement result, the light transmittance (%) at 567 nm was determined.

The results thereof are shown in Table 1.

As described above, the lower the light transmittance (%) at 567 nm, the superior is the dyeability of the resin formed body.

**[Table 1]**

| | | | Example 1 | Example 2 | Example 3 | Example 4 | Example 5 | Example 6 |
|---|---|---|---|---|---|---|---|---|
| Polythiol composition | Peak area of compound C2 with respect to a total peak area of 100 of compound C2 and main component (polythiol component A1) | | 1.650 | 2.500 | 3.130 | 5.100 | 8.170 | 12.830 |
| | Peak area of compound C3 with respect to a total peak area of 100 of compound C3 and main component (polythiol component A1) | | 0.170 | 0.490 | 0.610 | 1.170 | 2.000 | 3.310 |
| | Peak area of compound C4 with respect to a total peak area of 100 of compound C4 and main component (polythiol component A1) | | 0.070 | 0.090 | 0.102 | 0.280 | 0.547 | 1.040 |
| Resin formed body | Yellowness | YI | 1.68 | 1.70 | 1.80 | 2.03 | 2.14 | 2.23 |
| | Dyeability | Light transmittance (%) Wavelength: 567 nm | 39.12 | 39.05 | 39.11 | 39.03 | 38.66 | 36.77 |

As shown in Table 1, resin formed bodies having excellent dyeability were obtained in Examples 1 to 6 where a polythiol composition, which contained the compound C2 having a retention time of from 16.0 minutes to 19.0 minutes in a high-performance liquid chromatography measurement performed under the measurement conditions A and in which the compound C2 in the high-performance liquid chromatography measurement had a peak area of 1.000 or more with respect to a total of 100 of a peak area of the compound C2 and a peak area of the main component of the polythiol composition, was used.

The disclosure of Japanese Patent Application No. 2022-010749 filed on January 27, 2022 is hereby incorporated by reference in its entirety.

All the documents, patent applications and technical standards that are described in the present specification are hereby incorporated by reference to the same extent as if each individual document, patent application or technical standard is concretely and individually described to be incorporated by reference.

## Claims

1. A polythiol composition, comprising a polythiol compound,
wherein:
the polythiol composition comprises a compound C2 that has a retention time of from 16.0 minutes to 19.0 minutes in a high-performance liquid chromatography measurement performed under the following measurement conditions A, and
in the high-performance liquid chromatography measurement, the compound C2 has a peak area of 1.000 or more with respect to a total of 100 of a peak area of the compound C2 and a peak area of a main component of the polythiol composition:
-Measurement Conditions A-
MIGHTYSIL (registered trademark) RP-18 GP manufactured by Kanto Chemical Co., Inc. (particle size S: 5 µm, column shape: Φ6 mm × 150 mm, product number: 25477-96) is used as a column;
a mixed solution of acetonitrile/0.01 mol/L-aqueous potassium dihydrogen phosphate solution = 60/40 (vol/vol) is used as a mobile phase;
a mixed solution of 160 mg of the polythiol composition and 10 mL of acetonitrile is used as a measurement solution;
a UV detector having a measurement wavelength of 230 nm is used as a detector;
a column temperature is set at 40°C;
a flow rate is set at 1.0 mL/min; and
an injection amount is set at 2 µL.

2. The polythiol composition according to claim 1, wherein the compound C2 is a polythiol compound having a molecular weight of 320.

3. The polythiol composition according to claim 1 or 2,
wherein:
the polythiol composition further comprises a compound C3 that has a retention time of from 32.0 minutes to 35.0 minutes in the high-performance liquid chromatography measurement, and
in the high-performance liquid chromatography measurement, the compound C3 has a peak area of 0.050 or more with respect to a total of 100 of a peak area of the compound C3 and the peak area of the main component of the polythiol composition.

4. The polythiol composition according to claim 3, wherein the compound C3 is a polythiol compound having a molecular weight of 426.

5. The polythiol composition according to any one of claims 1 to 4,
wherein:
the polythiol composition further comprises a compound C4 that has a retention time of from 21.5 minutes to 25.0 minutes in the high-performance liquid chromatography measurement, and
in the high-performance liquid chromatography measurement, the compound C4 has a peak area of 0.030 or more with respect to a total of 100 of a peak area of the compound C4 and the peak area of the main component of the polythiol composition.

6. The polythiol composition according to claim 5, wherein the compound C4 is a polythiol compound having a molecular weight of 380.

7. The polythiol composition according to any one of claims 1 to 6, wherein the main component is a polythiol compound (XA) comprising three or more mercapto groups.

8. The polythiol composition according to claim 7, wherein the polythiol compound (XA) is a polythiol component A1 that is 4-mercaptomethyl-1,8-dimercapto-3,6-dithiaoctane, or a polythiol component A2 that is at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

9. The polythiol composition according to claim 7, wherein polythiol compound (XA) is a polythiol component A1 that is 4-mercaptomethyl-1,8-dimercapto-3,6-dithiaoctane.

10. A polymerizable composition for an optical material, comprising:
a polyiso(thio)cyanate compound; and
the polythiol composition according to any one of claims 1 to 9.

11. The polymerizable composition for an optical material according to claim 10, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

12. The polymerizable composition for an optical material according to claim 10 or 11, comprising a polyiso(thio)cyanate composition comprising the polyiso(thio)cyanate compound.

13. The polymerizable composition for an optical material according to claim 12, wherein:
the polyiso(thio)cyanate composition comprises:
xylylene diisocyanate; and
at least one selected from the group consisting of the following compounds (N1), (N2), and (N3),
when the polyiso(thio)cyanate composition comprises the compound (N1), the compound (N1) has a peak area of 0.20 ppm or more with respect to a peak area of xylylene diisocyanate of 1 in a gas chromatography measurement performed under the following GC conditions 1,
when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
-GC Conditions 1-
Filler: DB-1 (film thickness) 1.5 µm
Column: 0.53 mm in inner diameter × 60 m in length (manufactured by Agilent Technologies, Inc.)
Oven temperature: increased from 130°C to 220°C at 3°C/min, and further increased to 300°C at 10°C/min after reaching 220°C
Split ratio: pulsed splitless method
Inlet temperature: 280°C
Detector temperature: 300°C
Carrier gas: N₂ 158 kPa, H₂ 55 kPa, Air 45 kPa (constant pressure control)
Solvent: chloroform
Sample concentration: 2.0%-by-mass chloroform solution
Injection amount: 2 µL
Detection method: FID
- GC Conditions 2-
Column: HP-50+, 0.25 mm in inner diameter × 30 m in length × 0.25 µm in film thickness (manufactured by Hewlett-Packard Company)
Oven temperature: increased from 50°C to 280°C at 10°C/min, and held for 6 minutes after reaching 280°C
Split ratio: pulsed splitless method
Inlet temperature: 200°C
Detector temperature: 280°C
Carrier gas: He
Carrier gas flow rate: 1.0 ml/min (constant flow rate control)
Sample concentration: 1.0%-by-mass dichloromethane solution
Injection amount: 1.0 µL
Detection method: SIM (monitoring ion: m/z 180, 215) (content ratio of xylylene diisocyanate)

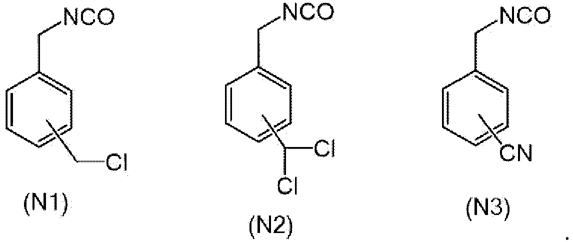

14. A resin, which is a cured product of the polymerizable composition for an optical material according to any one of claims 10 to 13.

15. A formed body, comprising the resin according to claim 14.

16. An optical material, comprising the resin according to claim 14.

17. A lens, comprising the resin according to claim 14.

18. A method of producing a polymerizable composition for an optical material, the method comprising a step of mixing a polyiso(thio)cyanate composition comprising a polyiso(thio)cyanate compound with the polythiol composition according to any one of claims 1 to 9.

19. The method of producing a polymerizable composition for an optical material according to claim 18, wherein:
the polyiso(thio)cyanate composition comprises:
xylylene diisocyanate; and
at least one selected from the group consisting of the following compounds (N1), (N2), and (N3),
when the polyiso(thio)cyanate composition comprises the compound (N1), the compound (N1) has a peak area of 0.20 ppm or more with respect to a peak area of xylylene diisocyanate of 1 in a gas chromatography measurement performed under the following GC conditions 1,
when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
- GC Conditions 1-
Filler: DB-1 (film thickness) 1.5 µm
Column: 0.53 mm in inner diameter × 60 m in length (manufactured by Agilent Technologies, Inc.)
Oven temperature: increased from 130°C to 220°C at 3°C/min, and further increased to 300°C at 10°C/min after reaching 220°C
Split ratio: pulsed splitless method
Inlet temperature: 280°C
Detector temperature: 300°C
Carrier gas: N₂ 158 kPa, H₂ 55 kPa, Air 45 kPa (constant pressure control)
Solvent: chloroform
Sample concentration: 2.0%-by-mass chloroform solution
Injection amount: 2 µL
Detection method: FID
- GC Conditions 2-
Column: HP-50+, 0.25 mm in inner diameter × 30 m in length × 0.25 µm in film thickness (manufactured by Hewlett-Packard Company)
Oven temperature: increased from 50°C to 280°C at 10°C/min, and held for 6 minutes after reaching 280°C
Split ratio: pulsed splitless method
Inlet temperature: 200°C
Detector temperature: 280°C
Carrier gas: He
Carrier gas flow rate: 1.0 ml/min (constant flow rate control)
Sample concentration: 1.0%-by-mass dichloromethane solution
Injection amount: 1.0 µL
Detection method: SIM (monitoring ion: m/z 180, 215) (content ratio of xylylene diisocyanate)

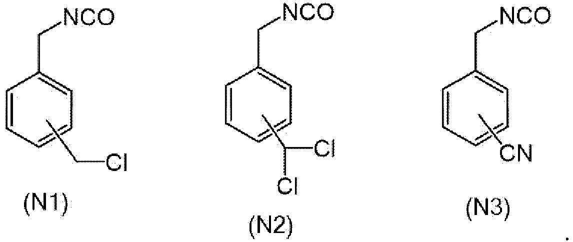

20. A composition set used for production of a polymerizable composition for an optical material, the composition set comprising:
a polyiso(thio)cyanate composition comprising a polyiso(thio)cyanate compound; and
the polythiol composition according to any one of claims 1 to 9.

21. The composition set according to claim 20, wherein:
the polyiso(thio)cyanate composition comprises:
xylylene diisocyanate; and
at least one selected from the group consisting of the following compounds (N1), (N2), and (N3),
when the polyiso(thio)cyanate composition comprises the compound (N1), the compound (N1) has a peak area of 0.20 ppm or more with respect to a peak area of xylylene diisocyanate of 1 in a gas chromatography measurement performed under the following GC conditions 1,
when the polyiso(thio)cyanate composition comprises the compound (N2), the compound (N2) has a peak area of 0.05 ppm or more with respect to a peak area of xylylene diisocyanate of 1 in a gas chromatography measurement performed under the following GC conditions 2, and
when the polyiso(thio)cyanate composition comprises the compound (N3), the compound (N3) has a peak area of 0.10 ppm or more with respect to a peak area of xylylene diisocyanate of 1 in a gas chromatography measurement performed under the following GC conditions 1:
- GC Conditions 1-
Filler: DB-1 (film thickness) 1.5 µm
Column: 0.53 mm in inner diameter × 60 m in length (manufactured by Agilent Technologies, Inc.)
Oven temperature: increased from 130°C to 220°C at 3°C/min, and further increased to 300°C at 10°C/min after reaching 220°C
Split ratio: pulsed splitless method
Inlet temperature: 280°C
Detector temperature: 300°C
Carrier gas: N₂ 158 kPa, H₂ 55 kPa, Air 45 kPa (constant pressure control)
Solvent: chloroform
Sample concentration: 2.0%-by-mass chloroform solution
Injection amount: 2 µL
Detection method: FID
- GC Conditions 2-
Column: HP-50+, 0.25 mm in inner diameter × 30 m in length × 0.25 µm in film thickness (manufactured by Hewlett-Packard Company)
Oven temperature: increased from 50°C to 280°C at 10°C/min, and held for 6 minutes after reaching 280°C
Split ratio: pulsed splitless method
Inlet temperature: 200°C
Detector temperature: 280°C
Carrier gas: He
Carrier gas flow rate: 1.0 ml/min (constant flow rate control)
Sample concentration: 1.0%-by-mass dichloromethane solution
Injection amount: 1.0 µL
Detection method: SIM (monitoring ion: m/z 180, 215) (content ratio of xylylene diisocyanate (XDI))
